# EUROPÄISCHE PATENTSCHRIFT

(11) **EP 0 269 935 B1**
(45) Veröffentlichungstag und Bekanntmachung des Hinweises auf die Patenterteilung: **12.01.1994**
(21) Anmeldenummer: 87116854.8
(22) Anmeldetag: 14.11.1987
(51) Int. Cl.: A61B 17/28, A61F 2/46, A61B 17/02

(54) **Zange zum Spreizen von Wirbelsäulenkörpern**
Expander forcep for the vertabra
Pince d'écortement pour les vertèbres

(30) Priorität: 05.03.1987 DE 3707097; 05.12.1986 DE 3641279
(43) Veröffentlichungstag der Anmeldung: 08.06.1988
(73) Patentinhaber: ESKA medical GmbH & Co., D-23556 Lübeck (DE)
(72) Erfinder: Grundei, Hans, D-2400 Lübeck (DE)
(74) Vertreter: Weiss, Christian, Dipl.-Ing.

(56) Entgegenhaltungen:
- CH-A- 285 617
- DE-U- 8 016 889
- US-A- 3 486 505

## Beschreibung

Die Erfindung bezieht sich auf eine Zange zum Auseinanderspreizen benachbarter Wirbelsäulenkörper gemäß dem Oberbegriff der beiden nebengeordneten Ansprüche 1 und 2.

Beim Auftreten von Schäden an Wirbelkörpern oder an den Bandscheiben der Wirbelsäule werden benachbarte Wirbelkörper zueinander durch metallische Implantate festgelegt oder die Bandscheibe ganz oder teilweise entfernt, und dann werden die entfernten Teile durch Implantate ersetzt. Um diese durchführen zu können, werden benachbarte Wirbelkörper auseinandergespreizt. Zum Spreizen solcher benachbarter Wirbelkörper hat man bisher einfache Zangen verwendet, deren Maulschenkel sich durch Zusammendrücken der Griffschenkel mit einem von der Schwenkachse der Zange ausgehenden Winkel öffnen. Dadurch konnten praktisch nur die Enden der Maulschenkel punktförmig an den einander zugekehrten Flächen benachbarter Wirbelkörper angreifen, so daß benachbarte Wirbelkörperflächen einen nach hinten offenen Winkel bildeten, der sodann mit einem Implantat ausgefüllt wurde. Die sich dabei ergebende Kipp- oder Winkellage der Wirbelkörper konnte zu Schäden der weiteren Wirbelkörper und unter Umständen auch zu Schmerzen für den Patienten führen.

Eine Zange mit den Merkmalen des Oberbegriffs des Anspruchs 1, wenngleich diese nicht zum Auseinanderspreizen benachbarter Wirbelsäulenkörper, sondern vielmehr zur Spreizung eines Distanzstückes für einen prothetischen Wirbelkörperersatz dient, zeigt die DE-U-8016889.

Darin ist beschrieben eine Zange, bei der die parallelen Dorne mittels einer Schere nach Art der Nürnberger Schere parallel voneinander wegbewegbar sind, wobei die an dem Distanzstück angreifenden Dorne dieses auseinanderziehen.

Für den direkten Ansatz dieser Zange an Wirbelsäulenkörper eignet sich die Zange gemäß der besagten Druckschrift nicht. Die Nürnberger Schere bietet hierzu nicht die notwendige Stabilität. Hierzu sei bemerkt, daß die Kraft zum Auseinanderspreizen benachbarter Wirbelkörper in der menschlichen Wirbelsäule beträchtlich ist.

Vor diesem Hintergrund ist es die Aufgabe der vorliegenden Erfindung, eine Zange der eingangs erwähnten Art so weiterzubilden, daß diese Zange hinreichend stabil ist für ein Auseinanderspreizen benachbarter Wirbelkörper, wobei die einander zugekehrten Flächen dieser Wirbelkörper beim Spreizen stets parallel oder im wesentlichen parallel zueinander bleiben.

Diese Aufgabe der Erfindung wird dadurch gelöst, daß bei der eingangs erwähnten Zange der eine Maulschenkel am freien Ende mit einem den einen Dorn aufnehmenden Träger starr verbunden ist, der senkrecht zur Dornrichtung eine Führung aufweist, an der der mit dem anderen Maulschenkel gelenkig verbundene und den zweiten Dorn aufweisende Träger geführt ist.

Gemäß einer Alternativlösung ist vorgesehen, daß der starr mit dem einen Maulschenkel verbundene bzw. verbindbare Dornträger mit einer zur Dornrichtung senkrechten Durchbohrung als Führung für eine am einen Ende durch ein Loslager mit dem zweiten Maulschenkel verbundene, unverdrehbare Schiene versehen ist, die am anderen Ende mit dem Träger für den zweiten Dorn versehen ist.

Durch die Lösung nach der Erfindung ist es erforderlich, benachbarte Wirbelsäulenkörper je mit einer Bohrung senkrecht zur Wirbelsäule zu versehen und die beiden Dorne der Zange in diese Bohrungen einzuführen, worauf die benachbarten Wirbelkörper durch Zusammendrücken der Zangengriffschenkel und dadurch eintretendes Spreizen der Maulschenkel und damit erfolgendes paralleles Auseinanderspreizen der beiden Dorne mit den einander zugekehrten Flächen parallel zueinander auseinander gespreizt werden, so daß die beschädigte Bandscheibe entfernt und durch ein Metallimplantat, vorteilhaft ein poriges Metallimplantat, ersetzt werden kann, welch letzteres eine innige feste Verbindung mit dem knöchernen Wirbelkörper eingeht und damit auch die benachbarten Wirbelkörper fest und sicher miteinander verbindet.

Die Zange nach der Erfindung mit weiteren vorteilhaften Merkmalen wird nachstehend anhand zweier Ausführungsbeispiele der Zeichnung erläutert. Es zeigen:
- Figur 1: eine Seitenansicht einer Spreizzange nach einem ersten Beispiel der Erfindung,
- Figur 2: eine Aufsicht auf die Zange nach Figur 1,
- Figur 3: einen senkrechten Schnitt nach Linie III bis III der Figur 1,
- Figur 4: eine Seitenansicht einer Spreizzange nach einem zweiten Beispiel der Erfindung,
- Figur 5: eine Aufsicht auf die Zange nach Figur 4,
- Figuren 6 und 7: zwei austauschbare Träger mit den parallelen Dornen in Seitenansicht.

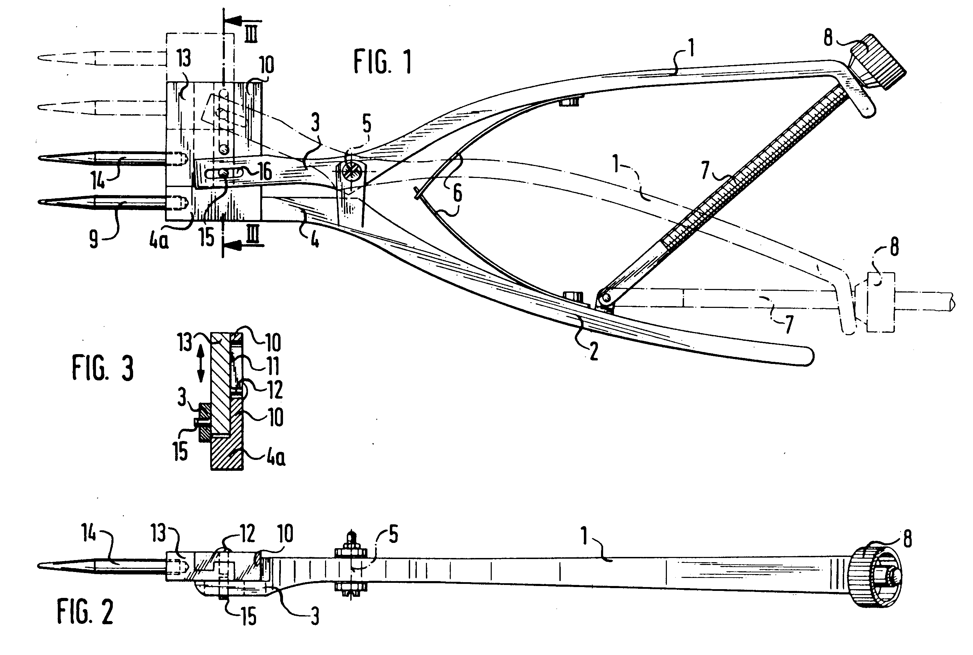

Die Zange zum Spreizen benachbarter Wirbelsäulenkörper besteht nach dem Beispiel Figur 1 bis 3 aus den beiden Griffschenkeln 1,2 und den beiden Maulschenkeln 3,4, die um die Achse 5 verschwenkbar sind. Die Griffschenkel 1,2 werden durch eine Feder 6 auseinandergespreizt und dabei werden die Maulschenkel 1,2 gegeneinander bewegt. Mit dem Griffschenkel 2 ist eine Schraube 7 gelenkig verbunden, die eine Bohrung des anderen Griffschenkels 1 durchgreift und auf deren Ende eine Mutter 8 aufschraubbar ist, durch deren Verschraubung die mehr oder weniger stark einander genäherten Schenkel 1,2 in ihrer Lage gegen den Druck der Feder 6 festgehalten werden.

Der Maularm 4 ist mit einem Träger 4a versehen, der am freien Ende eine Bohrung zur Aufnahme eines Dornes 9 besitzt. Der Träger 4a ist mit einer Führung 10 senkrecht zur gedacht verlängerten Achse des Dornes 9 versehen, die einen Langschlitz 11 besitzt, der von einem Zapfen 12 eines an der Führung 10 gleitenden Trägers 13 durchgriffen ist. Der Träger 13 ist auf der freien Endfläche mit mehreren Bohrungen zum Einsetzen eines zweiten Dornes 14 parallel zum Dorn 9 versehen.

Seitlich ist der Träger 13 mit einem Mitnehmerzapfen 15 versehen, der einen Langschlitz 16 des Maulschenkels 3 durchgreift. Durch diese Verbindung wird der Träger 13 beim Zusammendrücken der Griffschenkel 1,2 an der Führung 10 verschoben, wobei die parallelen Dorne 9, 14 eine Abstandslage einnehmen, in der sie in vorher vorgenommene parallele Bohrungen benachbarter Wirbelkörper für die Entfernung der Bandscheibe oder Bandscheibenteile oder zur Behebung sonstiger Schäden auseinanderspreizen, werden die Griffschenkel 1,2 sodann entsprechend weiter zusammengedrückt, und danach in dieser Lage durch die auf der Schraube 7 verschraubte Mutter 8 festgehalten, so daß der Mediziner das Entfernen der Bandscheibe oder von Teilen der Bandscheibe bequem durchführen und anschließend starre, vorteilhaft poröse Metallimplatate als Ersatz implantieren kann.

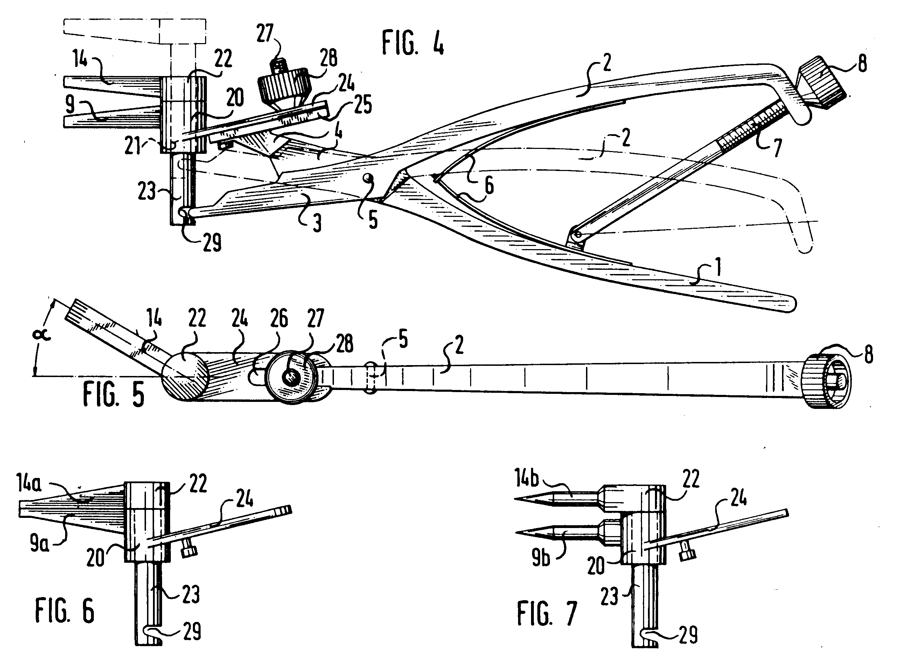

Nach den zweiten, sehr vorteilhaften Beispiel der Erfindung nach den Figuren 4 bis 7 sind die Träger mit den Dornen vereinfacht und austauschbar ausgebildet. In diesem Fall ist der Träger 4a nach Figur 1 durch einen Träger 20 ersetzt, der mit einer von oben nach unten durchlaufenden Durchbohrung 21 senkrecht zur Richtung des Dornes 9 versehen ist, wobei durch die Bohrung axial verschiebbar aber unverdrehbar eine mit dem zweiten Träger 22 verbundene, geführte Schiene 23 verläuft. Es ist dabei der Träger 20 des Dornes 9 auf der Seite der Zangenmaulschenkel 3,4 mit einem Arm 24 starr verbunden, der mit einer mit dem Maulschenkel 4 versehene Konsole 24 verbindbar ist. Der Arm 24 besitzt hierzu einen zum Ende offenen Längsschlitz 26, der beim Aufschieben des Armes 24 auf die Konsole 25 gemeinsam mit den Trägern 9,14 eine Schraube 27 einfaßt, auf der eine Mutter 28 zur festen Verbindung der beiden Teile 24 und 25 aufschraubbar ist.

Die Schiene 23 des Trägers 22 für den Dorn 14 besitzt am Unterende eine längliche Ausnehmung 29, in die das Ende des Maulschenkels 3 beim Aufschieben des Armes 24 mit den Trägern 20,22 auf die Konsole lose eingreift. Durch Zusammendrücken der Griffschenkel 1,2 gegen die Feder 6, durch die die Träger 20,22 in Anlage gehalten werden, werden die Maulschenkel 3,4 und damit die Träger 20, 22 mit den Dornen 9, 14 auseinandergespreizt, wie schon zu Figuren 1 bis 3 beschrieben wurde.

Um beim Auseinanderspreizen zweier benachbarter Wirbelkörper die Sicht für den Arzt durch die Griffschenkel 1,2 nicht zu behindern, ist es vorteilhaft, die beiden Dorne 9,14 in einer Ebene anzuordnen, die mit der Ebene der Zangenschenkel 1,2 bzw. 3, 4 einen Winkel alpha bildet, wie sich aus Figur 5 ergibt.

Durch die lösbare Verbindung der Dornenträger 20, 22 mit den Maulschenkeln 3,4 der Zange ist es möglich, die Dornträger durch Träger 20,22 mit abgeänderten Dornen 9a, 9b und 14 a, 14b entsprechend des Figuren 6 bis 7 zu ersetzen, wenn dies für den jeweiligen Anwendungszweck günstig ist.

## Patentansprüche

1. Zange zum Auseinanderspreizen benachbarter Wirbelsäulenkörper durch Öffnen der Maulschenkel (3, 4) mittels der zusammendrückbaren Griffschenkel (1, 2), bei der mit den Maulschenkeln (3, 4) zwei parallele Dorne (9, 14) verbunden sind, von denen der eine beim Spreizen der Maulschenkel (3, 4) durch eine Führung (10, 11) parallel zum anderen Dorn verschiebbar ist,
dadurch gekennzeichnet, daß
der eine Maulschenkel (4) am freien Ende mit einem den einen Dorn (9) aufnehmenden Träger (4a) starr verbunden ist, der senkrecht zur Dornrichtung eine Führung (10) aufweist, an der ein mit dem anderen Maulschenkel (3) gelenkig verbundener und den zweiten Dorn (14) aufweisender Träger (13) geführt ist.

2. Zange zum Auseinanderspreizen benachbarter Wirbelsäulenkörper durch Öffnen der Maulschenkel (3, 4) mittels der zusammendrückbaren Griffschenkel (1, 2), bei der mit den Maulschenkeln (3, 4) zwei parallele Dorne (9, 14) verbunden sind, von denen der eine beim Spreizen der Maulschenkel (3, 4) durch eine Führung (21, 23) parallel zum anderen Dorn verschiebbar ist,
dadurch gekennzeichnet,
daß ein starr mit dem einen Maulschenkel (4) verbundener bzw. verbindbarer Dornträger (20) mit einer zur Dornrichtung senkrechten Durchbohrung (21) als Führung für eine am einen Ende durch ein Loslager (29) mit dem zweiten Maulschenkel (3) verbundene, unverdrehbare Schiene (23) versehen ist, die am anderen Ende mit einem Träger (22) für den zweiten Dorn (14) versehen ist.

3. Zange nach Anspruch 1, dadurch gekennzeichnet, daß der gelenkig mit dem zweiten Maulschenkel (3) verbundene Träger (13) mit einem seitlichen Zapfen (15) versehen ist, der durch einen Längsschlitz (16) des Maulschenkels (3) greift.

4. Zange nach Anspruch 2, dadurch gekennzeichnet, daß die beiden Dornträger (20, 22) austauschbar mit den beiden Maulschenkeln (3, 4) verbindbar sind.

5. Zange nach Anspruch 2 und 4, dadurch gekennzeichnet, daß der eine mit der Führungsbohrung (21) versehene Dornträger (20) durch einen seitlichen Arm (24) mit einer Konsole (25) des zugehörigen Maulschenkels (4) starr verbindbar ist.

6. Zange nach einem der Ansprüche 1 bis 5, dadurch gekennzeichnet, daß die beiden Dorne (9, 14) in einer Ebene liegen, die eine Winkellage (alpha) zur Ebene durch die beiden Griffschenkel (1, 2) der Zange einnimmt.

7. Zange nach Anspruch 2 oder nach einem der Ansprüche 4 bis 6, dadurch gekennzeichnet, daß die beiden Dornträger (20, 22) mit mehr als zwei Bohrungen, vorteilhaft Gewindebohrungen, zum wahlweisen Einsetzen je zweier Dorne (9, 14) im unterschiedlichen Abstand voneinander versehen sind.

8. Zange nach einem der Ansprüche 1 bis 7, dadurch gekennzeichnet, daß an den einen Griffschenkel (2) eine Schraube (7) gelenkig angreift, die eine Bohrung des anderen Griffschenkels (1) frei durchgreift und auf deren Ende eine Mutter (8) verschraubbar ist, durch die die Griffschenkel (1, 2) gegen eine das Spreizen der Griffschenkel ( 1, 2) bewirkende Feder (6) in einer zusammengedrückten Lage feststellbar sind.

## Claims

1. Forceps to spread apart adjacent vertebrae by opening the jaw (3, 4) with the aid of the pressable handle legs (1, 2), at which forceps two parallel mandrels (9, 14) are connected with the jaw legs (3, 4) one of which being deplacable by a guide (10) in parallel direction to the upper mandrel when the jaw legs are spread apart,
characterized in that
one of the jaw legs (4) is rigidly connected at its free end with a bracket (4a) which integrates one of the mandrels (9) and which comprises a guide (10) in perpendicular orientation to the direction of the mandrel, along which guide a bracket (13) is guided being swivel-mounted to the upper jaw (3) and comprising the second mandrel (14).

2. Forceps to spread apart adjacent vertebrae by opening the jaw legs (3, 4) with the aid of the pressable handle legs (1, 2), at which forceps two parallel mandels (9, 14) are connected with the jaw legs (3, 4), one of which being replaceable by a guide (10) in parallel orientation to the other mandel when the jaw legs are spread apart,
characterized in that
a mandrel bracket (20) being rigidly connected with one of the jaw legs (4) or rigidly connectable thereto is provided with a bore (21) in perpendicular orientation to the direction of the mandel and serving a as a guide for a non-distortable fishplate (23) connected at its one end with the second jaw leg (3) by a movable bearing (29), said fishplate being provided at the other end with a bracket (22) for the second mandrel (14).

3. Forceps according to claim 1, characterized in that the bracket (13) swivel-mounted at the second jaw leg (3) is provided with a lateral pilot (15) which reaches through a longitudinal slip (16) of the jaw leg (3).

4. Forceps according to Claim 2, characterized in that the two mandrel brackets (20, 22) are connectable with the two jaw legs (3, 4) interchangeably.

5. Forceps according to claims 2 and 4, characterized in that the one mandrel bracket (20) provided with the guide bore (21) is rigidly connectable with a console (25) of the appropriate jaw leg (4) by means of a lateral arm (24).

6. Forceps according to one of claims 1 to 5, characterized in that the two mandrels (9, 14) lay in a plane having an angular orientation (alpha) with respect to the plane through the two handle legs (1, 2) of the forceps.

7. Forceps according to claim 2 or according to one of Claims 4 to 6, characterized in that the two mandrel brackets (20, 22) are provided with more then two bores, preferably tapped bores, in order to be in the condition to optionally insert each two mandrels (9, 14) having different distances one from another.

8. Forceps according to one of claims 1 to 7, characterized in that into one of the handle legs (2) a screw (7) engages in articulated manner which screw freely penetrates a bore of the other handle leg (1) and onto the end of which, a nut (8) can be screwed by which the handle legs (1, 2) can be screwed in pressed position against a spring (6) effecting the spreading of the handle legs (1, 2).

## Revendications

1. Pince pour écarter des vertèbres voisines par ouverture des becs (3, 4) de la pince au moyen de ses branches (1, 2) approchables l'une de l'autre par compression manuelle, sur laquelle deux broches parallèles (9, 14) sont reliées aux becs (3, 4), dont l'une est déplaçable parallèlement à l'autre broche par un guide (10, 11) lors de l'écartement des becs (3, 4),
caractérisée en ce que
l'un des becs (4) est relié rigidement, à son extrémité libre, à un support (4a) portant une broche (9) et qui présente transversalement à la direction de la broche un guide (10) sur lequel est guidé un support (13) relié de façon articulée à l'autre bec (3) et portant la seconde broche (14).

2. Pince pour écarter des vertèbres voisines par ouverture des becs (3, 4) de la pince au moyen de ses branches (1, 2) approchables l'une de l'autre par compression manuelle, sur laquelle deux broches parallèles (9, 14) sont reliées aux becs (3, 4), dont l'une est déplaçable parallèlement à l'autre broche par un guide (21, 23) lors de l'écartement des becs (3, 4),
caractérisée en ce que
un support de broche (20) relié ou pouvant être relié rigidement à un bec (4), est pourvu d'un perçage traversant (21), perpendiculaire à la direction de la broche, en tant que guide pour une réglette (23) non rotative reliée à une extrémité par un palier libre (29) au second bec (3) et dont l'autre extrémité est pourvue d'un support (22) pour la seconde broche (14).

3. Pince selon la revendication 1, caractérisée en ce que le support (13) relié de façon articulée au second bec (3), est pourvu d'un tenon latéral (15) qui traverse une fente longitudinale (16) de ce bec (3).

4. Pince selon la revendication 2, caractérisée en ce que les deux supports de broches (20, 22) peuvent être reliés de façon interchangeable aux deux becs (3, 4).

5. Pince selon les revendications 2 et 4, caractérisée en ce que le support de proche (20) pourvu du perçage de guidage (21), peut être relié rigidement par un bras latéral (24) à une console (25) du bec (4) correspondant.

6. Pince selon une des revendications 1 à 5, caractérisée en ce que les deux broches (9, 14) sont situées dans un plan formant un angle alpha avec le plan contenant les deux branches (1, 2) de la pince.

7. Pince selon la revendication 2 ou selon une des revendications 4 à 6, caractérisée en ce que les deux supports de broches (20, 21) sont pourvus de plus de deux trous, de préférence de plus de deux trous taraudés, pour la mise en place sélective de chaque fois deux broches (9, 14) à des distances différentes l'une de l'autre.

8. Pince selon une des revendications 1 à 7, caractérisée en ce qu'une vis (7) attaque par une articulation l'une des branches (2) de la pince et traverse librement un trou de l'autre branche (1), vis sur l'extrémité de laquelle peut être vissé un écrou (8) par lequel les branches (1, 2) sont immobilisables, à l'encontre de la force d'un ressort (6) produisant l'écartement mutuel des branches (1, 2), à une position où les branches ont été approchées l'une de l'autre par compression manuelle.
